Europäisches Patentamt

(19) European Patent Office  (11) Numéro de publication : **0 128 103**

Office européen des brevets  **B1**

(12) **FASCICULE DE BREVET EUROPÉEN**

(45) Date de publication du fascicule du brevet :  (51) Int. Cl.⁴ : **A 61 N  1/04**, A 61 B  5/04,
11.10.89  A 61 N  1/22

(21) Numéro de dépôt : **84420095.6**

(22) Date de dépôt : **30.05.84**

(54) **Appareil équipant un vêtement pour l'émission ou la réception d'impulsions électriques.**

(30) Priorité : 01.06.83 US 499866
27.07.83 US 517810
22.05.84 US 611731

㊸ .Date de publication de la demande :
12.12.84 Bulletin 84/50

(45) Mention de la délivrance du brevet :
11.10.89 Bulletin 89/41

(84) Etats contractants désignés :
AT BE CH DE FR GB IT LI LU NL SE

(56) Documents cités :
FR–A–  429 322
FR–A– 1 420.114
FR–A– 1 429 342
FR–A– 2 342 082
US–A– 3 187 745
US–A– 3 556 105
US–A– 3 610 250
US–A– 4 121 575
US–A– 4 239 046

(73) Titulaire : **BIO-STIMU TREND CORPORATION**
**14851 N.W. 27th Avenue**
**Opa Locka Florida 33054 (US)**

(72) Inventeur : **Granek, Herman**
**230 174th St., Apt. 603**
**Miami Beach Florida 33160 (US)**
Inventeur : **Granek, Murry**
**488 Northwest 165th St. Rd., Apt. 411**
**Miami Florida 33169 (US)**
Inventeur : **Church, John**
**17345 N.W. 62 Court**
**Miami Florida 33015 (US)**

(74) Mandataire : **Dupuis, François**
**Cabinet Charras 3 Place de l'Hôtel-de-Ville**
**F-42000 St.Etienne (FR)**

EP 0 128 103 B1

## Description

Cette invention est un vêtement contenant des voies conductrices aux différentes parties du corps ayant des points de contact électrique avec la peau qui peuvent être choisis en appliquant un liquide électriquement conducteur par ces points présélectionnés dans le vêtement.

Divers procédés médicaux nécessitent la réception de signaux électriques de diverses parties du corps ou l'application de signaux électriques à d'autres points du corps. Parmi ces procédés, il y a la T.E.N.S. (simulation électrique transcutanée des nerfs), E.M.S. (stimulation musculaire électrique), F.E.S. (stimulation électrique fonctionnelle), E.M.G. (électromyogramme), E.E.G. (électroencéphalogramme) E.K.G. (électro-cardiogramme), la transmission de signaux engendrés par ordinateur pour le mouvement moteur stimulé chez les patients quadriplégiques ou paraplégiques, transmission de signaux à des endroits spécifiques pour la stimulation du développement des os transcutanée, et des systèmes d'éduction pour permettre l'identification immédiate et précise des points importants connus de l'anatomie pour l'envoi ou le contrôle de signaux biomédicaux. Les produits actuels à fixer sur le corps exigent des fils individuels et électrodes qui gênent le mouvement du corps et pendant les mouvements du corps les électrodes se débranchent souvent accidentellement. De nombreuses électrodes ne laissent pas la peau respirer ; les propriétés chimiques irritent parfois la peau nécessitant un soin spécial de la peau pendant de longues périodes de contrôle ou stimulation. La plupart des électrodes nécessitent l'élimination des poils ou cheveux pour une bonne fixation, et beaucoup de points sur le corps ne conviennent pas à la mise en place standard des électrodes en raison de leur configuration anatomique particulière, mouvement ou forme. Les dispositifs des techniques précédentes pour l'incorporation d'électrodes dans les vêtements se sont généralement limités à des chaussures comme dans le brevet U. S. 3.941.137 de Vredenbregt, et autres, ceintures comme dans le brevet U. S. 502.776 de Se Cheverall et autres, masques comme dans le brevet U. S. 3.279.468 de LeVine. Ces dispositifs sont limités à certaines zones du corps et ne peuvent pas être généralisés pour s'adapter à la plus grande partie du corps humain, y compris le torse, les jambes et les bras.

On cite également au titre de l'art antérieur selon l'article 54-2 de la CBE le brevet U. S. 3.610.250.

Un objet de cette invention est de fournir un moyen d'application d'électrodes à n'importe quelle partie du corps tout en permettant le mouvement du corps et en maintenant la fixation de ces électrodes. Un autre objet est d'assurer un moyen de maintien du fluide conducteur dans le vêtement aux points désignés sans tacher les vêtements extérieurs. Un autre objet est de prévoir un réservoir pour le fluide conducteur pour permettre une interface étendue.

Ces objectifs sont satisfaits en construisant un vêtement en tissu non-conducteur ordinaire. A titre d'illustration, un tel vêtement pourrait être un bas, un sweater à manches longues, un collant, des gants longs ou un masque. Une multitude de points sur le corps utilisés pour une application particulière sont marqués sur le vêtement comme points désignés. Ces points désignés sont électriquement reliés par des conducteurs à une prise pour fixation à un appareil électrique extérieur ou intérieur. Dans des vêtements pour le contrôle ou la stimulation, chaque point désigné a son propre conducteur. Dans d'autres vêtements pour la stimulation, chaque point désigné pourrait partager un des conducteurs en nombre relativement faible. Dans ce dernier cas, le point désigné pourrait être activé en appliquant un liquide conducteur au point sélectionné. Les conducteurs peuvent être un milieu conducteur tricoté ou tissé dans le tissu, des fils cousus sur le tissu ou du tissu conducteur cousu sur la base non-conductrice. Divers moyens d'accroissement peuvent être ajoutés à chaque point désigné. Par exemple des tubes qui pénètrent dans le vêtement peuvent être utilisés pour accélérer le fluide conducteur jusqu'à la peau. Des électrodes sur la surface intérieure peuvent également être appliquées à des fins spécifiques. L'électrode et les conducteurs sur la surface intérieure peuvent être isolés de la peau et créer un aspect esthétique différent. Les poches de tissu sur la surface intérieure ou extérieure peuvent faciliter l'application du fluide et protéger les vêtements extérieurs.

Selon l'invention, l'appareil équipant un vêtement pour délivrer ou recevoir des impulsions électriques comprend :
un vêtement fait d'une étoffe poreuse non conductrice électriquement et recevant extérieurement une pluralité d'électrodes reliées des conducteurs fixés à l'extérieur du vêtement de manière à recouvrir une partie de celui-ci ;
une extrémité des conducteurs étant en contact électrique sélectif avec un émetteur d'impulsions électriques ou un capteur électrique, les conducteurs étant positionnés par rapport aux électrodes de manière qu'une partie soit en contact électrique lorsque l'appareil est utilisé, caractérisé en ce qu'il comprend :
au moins une poche sur l'extérieur définie par le tissu du vêtement et un revêtement en matériau fixé audit tissu sur sa périphérie, une électrode étant disposée dans chaque poche en définissant un espace pour l'introduction d'une matière électrolytique ;
la porosité du vêtement étant suffisante pour permettre à ladite matière fluide de pénétrer à travers de manière que la peau du patient soit mouillée par ladite matière fluide lorsque le vêtement est porté afin de compléter la connexion électrique entre l'électrode et la peau du patient.

Ces caractéristiques et d'autres caractéristiques de fonctionnement et de construction de l'invention apparaîtront de façon plus évidente dans la description détaillée ci-après en se référant aux figures des plans d'accompagnement qui illustrent une réalisation préférée comme exemples non limitatifs.

La figure 1 montre le vêtement avec des points désignés multiples, chacun avec des conducteurs individuels.

La figure 2 montre le vêtement avec des points désignés multiples reliés à deux conducteurs seulement.

La figure 3 montre un point désigné avec une poche en plan.

La figure 4 montre le point désigné de la figure 3 en section.

La figure 5 montre une électrode isolée extérieure en section.

La figure 6 montre une capsule de fluide conducteur au point désigné en section.

La figure 7 est une vue en section transversale d'encore une autre réalisation d'un moyen à électrode en place.

La figure 8 est la réalisation de la figure 7 avec gel électrolytique en place.

La figure 9 est une vue en plan supérieure des réalisations 7 et 8.

La figure 10 est une vue en section transversale d'une autre réalisation d'un moyen à électrcde en place.

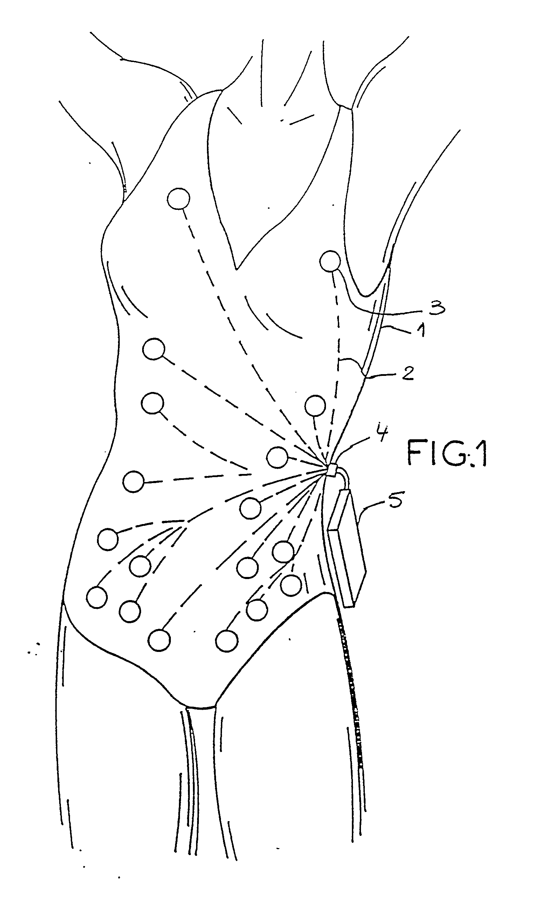

En se référant maintenant aux plans où les numéros de référence sont utilisés pour désigner des pièces sur toutes les diverses figures, on représente à la figure 1 un vêtement (1) couvrant le torse humain, dans lequel sont encastrés une multitude de conducteurs isolés (2) comme des fils ou fil conducteur. Une extrémité de chaque conducteur (2) se termine aux points désignés (3) et l'autre extrémité de chaque conducteur se termine dans un ou plusieurs connecteurs (4). Le connecteur (4) est utilisé pour relier le vêtement (1) à un dispositif extérieur (5) pour contrôler ou fournir des impulsions de stimulation. Le vêtement pourrait être construit de façon à couvrir le torse supérieur et les bras et pourrait comporter des gants solidaires ou un masque. Le vêtement pourrait être construit pour ne couvrir que le torse inférieur et les jambes comme un collant. Le vêtement pourrait être d'une pièce, couvrant le corps entier ou seulement ce que l'on désire. Le vêtement pourrait être fait de plusieurs parties portées en même temps et reliées extérieurement aux unités électriques en collaboration comme le dispositif extérieur (5).

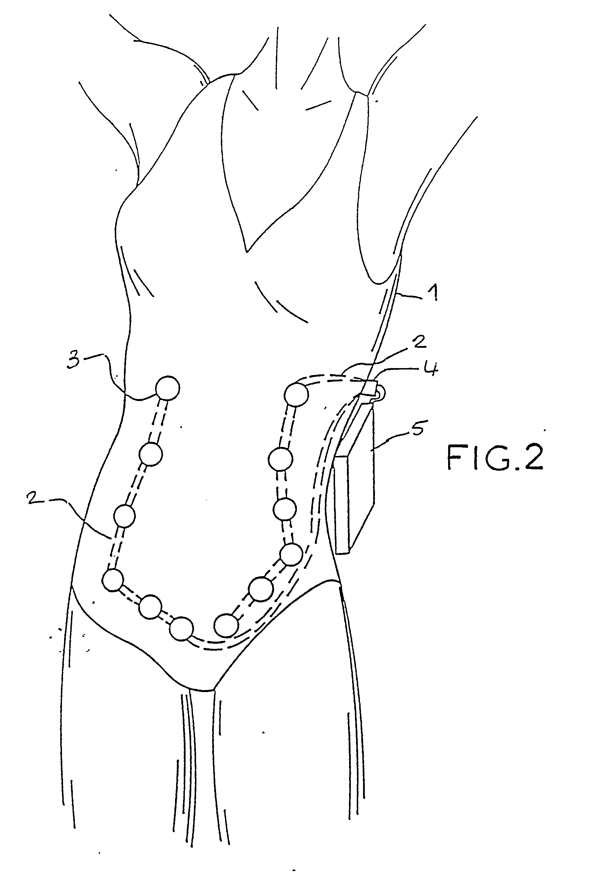

En se référant maintenant à la figure 2, le vêtement (1) est représenté avec les points désignés (3) reliés par des voies multiples par les conducteurs (2) sur l'extérieur du vêtement (1) au connecteur (4) et ensuite au dispositif extérieur (5).

Le vêtement (1) sera fait, d'une façon générale, de matières non conductrices, afin d'isoler les différentes trajectoires des conducteurs (2) les unes des autres et de la peau du patient. Les points désignés sélectionnés peuvent être faits de tissu conducteur à des fins spéciales.

Ce tissu conducteur pourrait être, par exemple, des produits conducteurs HI-MEG®, distribués par VELCRO®, USA INC. Les produits HI-MEG® sont conducteurs dans des plages de basse tension et basse intensité. Un facteur de résistance de 2 Ohms par pouce carré est le maximum qu'il est possible d'obtenir dans l'un des produits HI-MEG® ce qui fournit une méthode de charge statique effectuant des taux de fuite allant jusqu'à 100 % dans une matière standard si la surface est conductrice. En positionnant des épaisseurs supplémentaires de matière HI-MEG® en plusieurs endroits, sur une surface non-conductrice en une opération, la charge statique sera encore davantage réduite. Les produits HI-MEG® maintiennent leur efficacité à l'élimination de la charge statique à des niveaux d'humidité très bas (jusqu'à 0 %) et fonctionneront dans le vide pour réduire l'électricité statique sur les bandes d'enregistrement. Les produits HI-MEG® peuvent également être utilisés pour produire de l'électricité statique si on le souhaite. Le cyclage n'affecte pas la conductivité à moins que le revêtement argent ne soit excessif, provoquant un revêtement fragile qui peut se rompre à la flexion. Des essais complémentaires ont été faits pour s'assurer que les produits HI-MEG® continueront de fonctionner après une exposition et un entretien normaux. Les résultats sont résumés ci-après : (1) il peut y avoir de légères taches si la boucle HI-MEG® frotte contre une surface claire. (2) Les produits HI-MEG® peuvent être lavés à sec dans le commerce ou blanchis, mais beaucoup de détergents ou savons peuvent laisser un film qui réduira leur conductivité ; par conséquent, un bon rinçage est indispensable. (3) Bien que les produits HI-MEG® se ternissent en raison de leur teneur en argent, ce ternissage ne réduit pas leur efficacité. Une exception à cette règle est le ternissage produit dans un environnement riche en soufre. (4) la conductivité est réduite de moitié aux deux tiers après 48 h d'exposition au brouillard salin.

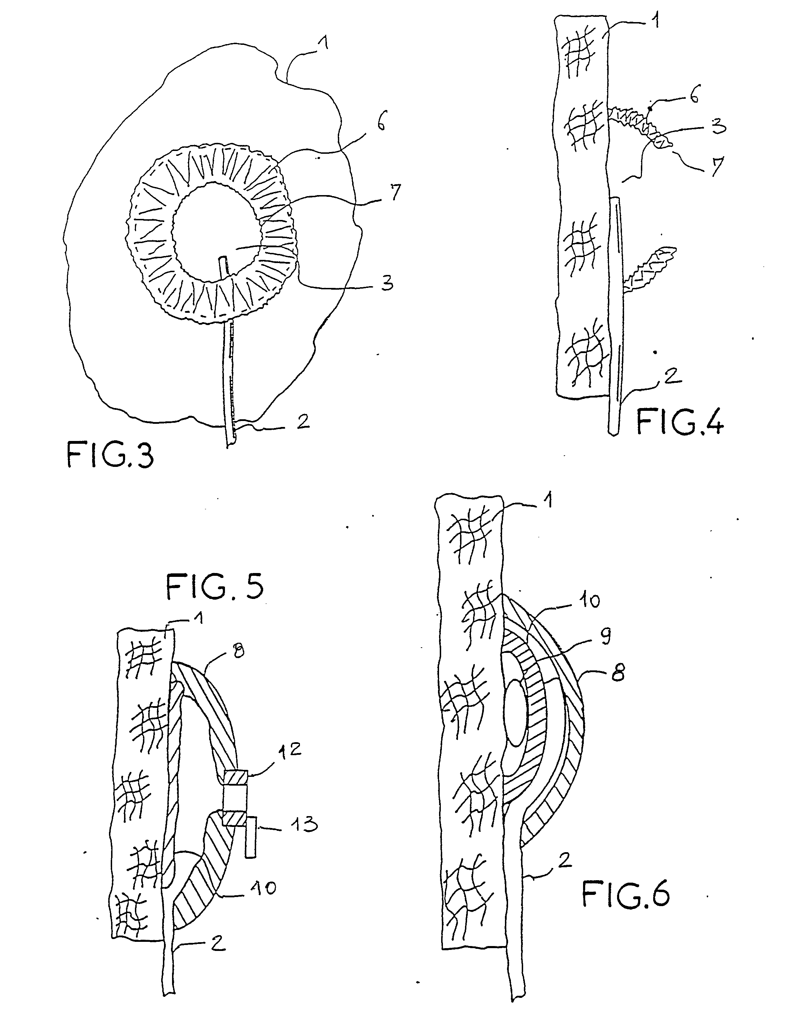

En se référant aux figures 3 et 4 des plans d'accompagnement, dans une autre réalisation le vêtement (1) a une poche en matière conductrice fixée au point désigné (3) sur la surface extérieure. Le conducteur (2) passe entre la surface du vêtement (1) et la poche (6). L'application d'un fluide conducteur dans cette poche (6) imprègne le vêtement (1) au point désigné (3) et établit le contact entre le conducteur (2) et la peau du porteur. La poche (6) aide à retenir le fluide conducteur. Ainsi l'application du fluide conducteur entraîne une activation ou sélection d'un point désigné (3) pour utilisation, ce qui supprime la nécessité de commutation extérieure comme dans la réalisation précédente.

En se référant maintenant à la figure 5 des plans d'accompagnement, le vêtement (1) est représenté avec l'électrode (10) sur l'extérieur du vêtement. Le conducteur (2) peut traverser le point désigné soit sur soit sous l'électrode (10). Un recouvrement à l'épreuve du fluide (8) formant poche avec le tissu du vêtement peut être placé sur l'électrode. L'orifice (12) avec chapeau (13) traverse le recouvrement à l'épreuve du fluide (8). Le fluide conducteur est appliqué par l'orifice (12), l'électrode (10) est reliée au conducteur (2) et à la peau du porteur au point désigné.

En se référant maintenant à la figure 6 des plans d'accompagnement, dans une autre réalisation, on représente une capsule de fluide conducteur (9) assemblée avec l'électrode (10). La capsule peut être sous ou sur l'électrode (10), peut-être sur l'intérieur du vêtement (1). La capsule (9) peut de même être montée sur l'intérieur du vêtement. Un revêtement à l'épreuve du fluide (8) peut être utilisé avec cette réalisation sans avoir besoin d'un orifice ou l'orifice peut être inclus pour assurer un moyen de renouvellement du fluide. La capsule peut être utilisée pour activer l'électrode en cassant la capsule par la pression des doigts ou en la piquant avec une aiguille ou un autre outil.

Les conducteurs (2), les électrodes (8), (10) et les poches (6) peuvent être en métal ou tissu conducteur comme HI-MEG®. Par exemple, dans une réalisation, le HI-MEG® est découpé en bandes à bords bruts de 9/16 de pouces. Il est introduit dans un accessoire de pliage qui, en liaison par exemple avec une machine à coudre classe 112, replie automatiquement la matière sur elle-même et amène la matière en haut du vêtement. Ce procédé forme un conducteur (2) d'une largeur d'environ 3/16". Les poches de sélection de fluide conducteur (6) sont utilisées en liaison avec le conducteur (2) et sont dimensionnées et placées suivant les besoins (ex. T.E.N.S., E.M.S., F.E.S., EKG, EEG, EMG, etc...). Chaque poche de stimulation et contrôle (6) emploie de la matière conductrice découpée à la cote voulue et à la forme voulue par exemple (1/32 pouce de circonférence à 2 × 6 pouces). Ces poches conductrices découpées (6) sont ensuite fixées au conducteur (2) à des points désignés anatomiquement (3), (T.E.N.S. : point d'acupuncture, points de déclenchement et dermatoses ; EMS, FES : points moteurs ; EKG : points standard de la poitrine et des extrémités, EMG : points EMG ; EEG : points EEG).

Lorsque le vêtement (1) est conçu pour un contrôle ou la stipulation d'endroits individuels, chaque point désigné (3) a son propre conducteur (2) comme à la figure 1. Un vêtement conçu pour la stimulation est construit avec des points désignés multiples (3) sur chaque conducteur (2) pour permettre une stimulation active à points multiples, ou comme à la figure 1 en variante, la polarité pour la stimulation sera d'une façon générale la moitié du vêtement (1) un pôle et la moitié opposée l'autre pôle. Le vêtement entier (1) peut être divisé de telle sorte que deux ou plusieurs générateurs d'impulsions puissent être montés, un pour la moitié supérieure et l'autre pour la moitié inférieure du vêtement. La matière de base du vêtement peut être en toute matière conductrice non électrique adéquate et construite de la façon habituelle.

Chaque poche (6) a par exemple un orifice d'application de 1/64 pouce à 1/2 pouce (7) sur chaque segment de 3/2 pouce, ce qui permet pour chaque application d'un fluide conducteur au vêtement (1) la pénétration ultérieure jusqu'à la peau sans avoir besoin de pression pour pousser le fluide dans chaque poche (6). Cet orifice d'application (7) empêche également le fluide conducteur d'avoir à être appliqué à la surface extérieure de la poche (6) ce qui peut provoquer des fuites sur tout le vêtement extérieur. Le vêtement (1) peut être porté sous un moule afin d'employer EMS, T.E.N.S., EMG, ou EKG. Dans ce cas, les orifices reliés par des tubes peuvent être moulés dans ou placés à travers le moule afin d'appliquer le fluide conducteur. Un liquide conducteur à long terme peut être placé à l'intérieur d'une poche pour être activé juste avant moulage. L'embout d'application du distributeur de fluide conducteur est spécialement conçu pour disperser une quantité égale de fluide conducteur par une petite ouverture ou des ouvertures en permettant une distribution égale du fluide conducteur au vêtement (1) pénétrant ultérieurement jusqu'à la peau du porteur. La poche (6) avec orifice d'application (7) peut être combinée avec le cylindre tubulaire (17) et/ou l'électrode (18) si on le souhaite. L'orifice d'application assure l'insertion non visuelle de l'électrolyte. Les voies de conduction peuvent également être extérieures au vêtement. Du tissu à crochets et à boucles peut être utilisé pour la fixation d'un tissu sur l'autre sans piqûre. Ce vêtement est distribué par VELCRO® USA, INC. Il est fait référence aux brevets US 2 717 437, 3 009 235, 3 417 440, 3 417 528, 3 461 513, et 3 708 382. Ce tissu conducteur à crochets et à boucles peut être utilisé pour relier les points désignés ayant des poches (6) faites de tissu conducteur à crochets et à boucles ce qui supprime la nécessité au moins partielle si ce n'est totale des conducteurs cousus ou collés sur le vêtement. Le connecteur à l'appareil électrique extérieur peut se terminer en une borne de tissu à crochets et à boucles pour faciliter l'interconnexion dans cette réalisation.

Les voies de conduction électrique du tissu conducteur cousues, collées, soudées ou fixées par tout autre moyen dans un vêtement peuvent également être utilisées pour d'autres fonctions que le contrôle du corps humain ou la stimulation du corps. Dans un costume spatial, des bandes de tissu conducteur peuvent être reliées à des appareils, commandes, climatisation, voyants et autres fonctions électriques au lieu de fil. Ces bandes de tissu sont plus souples que les fils et moins sujettes à la rupture.

Le tissu non-conducteur du vêtement peut être du nylon, Dacron® polyester, coton ou rayonne cellulose acétate ou non tissées ou des combinaisons des précédents. Un lycra® nylon de 1 once à 20 onces en poids serait adéquat, le vêtement doit être poreux.

Les voies de conduction peuvent être piquées sur la surface du vêtement, collées, agrafées, ou fixées par une méthode équivalente. La voie de conduction peut également être tricotée dans le tissu, en utilisant par exemple une machine à tricoter Jacquard.

Le trou dans la poche (6) peut être équipé d'un passe-fil pour aider à appliquer l'électrolyte. Le passe-fil peut être changé contre un bouton standard à pression avec un trou de 0,03 à 0,1 pouce placé au centre. Le bouton à pression pourrait ainsi servir de doublage du conduit d'électrolyte comme un bouton à pression pour la connexion facultative directe des fils. Des exemples de technologie similaire qui pourraient être utilisés en liaison avec la poche (6) sont représentés dans les brevets US 4 121 575, et 4 202 344. Le cylindre tubulaire (17) n'est pas limité à une section transversale circulaire, mais peut être elliptique ou autre en section transversale.

Le fluide conducteur peut aller de la plupart de l'eau potable qui contient suffisamment d'électrolyte pour être utilisable pour certaines applications de stimulation, jusqu'au gel d'électrode standard.

Un exemple de ce gel d'électrode standard serait le gel d'électrode Signagel® distribué par Parker Laboratories of Orange, New Jersey, ayant les propriétés suivantes : gel visqueux, clair, aqueux, salin, teinte vert clair. Composition chimique : polymer, humectants, agent tensio-actif, coloris certifié FDA, chlorure de sodium, conservateurs, et eau-ultra-violet-désionisée. Conservateur : propyl paraben et méthyle paraben. Viscosité : 180 000 à 260 000 cps (Viscosimètre Brookfield modèle RVT, broche T-C, 2,5 T/mn), gamme de pH : 5,4 à 6,4 (p-H mètre Beckman, modèle 3500). Conductivité : 25 000 à 45 000 micro-ohms. Stérilité : bactériostatique. Sécurité : irritation de la peau et des yeux évaluée par la méthode DRAIZE ; il a été conclu que le gel n'était pas irritant pour les yeux et la peau des lapins. Durée de conservation : indéfinie. Précautions : pas de manutention spéciale exigée. Nomenclature de Bruxelles : autres produits pharmaceutiques, 30.05, sous-article n° 541.9 (9). D'autres électrolytes pourraient être comme dans les brevets américains 2 8872 926, 3 528 408 et 3 607 788.

Le cylindre tubulaire (17) peut être en plastique ou caoutchouc dans la réalisation non conductrice. Dans la réalisation conductrice, le cylindre tubulaire (17) et l'électrode (18) peuvent être en métal ou en caoutchouc aux silicones.

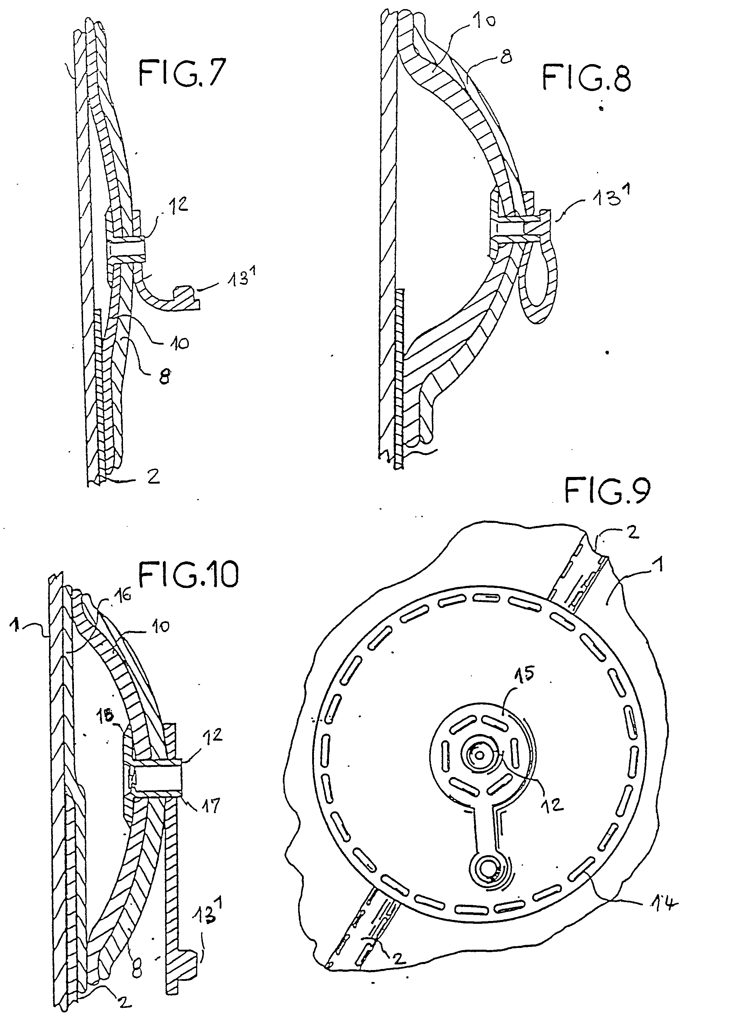

A la figure 7, on décrit une installation d'électrode qui dépeint une autre configuration pour l'ensemble. Par exemple, à l'endroit ou point où l'électrode est mise en place, l'électrode peut avoir une configuration semblable à un disque ou elle peut avoir toute autre configuration souhaitable depuis une forme oblongue jusqu'à une configuration de forme libre.

L'électrode (10) elle-même est construite en matière HI-MEG® mentionnée plus haut. Elle est recouverte d'une matière isolante (8) d'une surface, dimension et configuration couvrant l'électrode (10). L'ensemble est fixé le long d'une partie périphérique de celle-ci comme en cousant au vêtement (10) à cet endroit pour former une poche recevant le gel électrolytique définie par l'électrode (10) et une partie appropriée du vêtement. D'après la figure 9, on peut voir la couture cousue (14). L'électrode est cousue sur le vêtement (1) sur le conducteur (2) comme ceci est illustré.

L'orifice (12) dans cette réalisation est un élément tubulaire plastique court se terminant par une petite bride (12-1) qui constitue le moyen auquel l'électrode est fixée comme par couture. La figure 9 dépeint une couture (15) à cet effet.

Les figures 6 et 7 représentent le revêtement (13) pour l'orifice (12) comme étant un bouchon monté par friction séparé. Le bouchon a une partie d'obturation (13-1) qui se trouve à l'intérieur de l'orifice et a une partie plus grosse ayant un diamètre légèrement supérieur à l'orifice (12) pour assurer une prise par les ongles des doigts pour l'enlèvement à la main avant chargement de la poche avec le gel électrolytique et pour réinsertion par la suite.

Dans une autre modification démontrée par la figure 10, l'électrode (11) a un morceau de tissu (16) de matière similaire ou identique placé sur sa surface exposée et le vêtement. Le tissu (16) de matière similaire ou identique placé entre sa surface exposée et le vêtement. Le tissu (16) peut également être 100 % polyester. Il a une configuration semblable à l'électrode et est cousu sur le vêtement (1) lorsque l'électrode est cousue ou convenablement fixée sur celle-ci. Comme l'orifice communique intérieurement au-delà de l'électrode (10) mais pas par le tissu (16), la poche ainsi formée est définie par l'électrode (10) et le tissu (16). Ce dernier a la propriété de servir de mèche au gel électrolytique fourni à la poche dans le sens du vêtement (1) et à travers celui-ci. Le tissu (16) assure un effet de contrôle par lequel la migration du gel électrolytique au-delà de la surface définie par l'électrode est inhibée.

## Revendications

1. Appareil équipant un vêtement pour délivrer ou recevoir des impulsions électriques comprenant :
— un vêtement (1) fait d'une étoffe poreuse non conductrice électriquement et recevant extérieurement une pluralité d'électrodes reliées par des conducteurs (2) fixé à l'extérieur du vêtement de manière à recouvrir une partie de celui-ci ;
— une extrémité des conducteurs étant en contact électrique sélectif avec un émetteur d'impulsions électriques ou un capteur électrique, les conducteurs étant positionnés par rapport aux électrodes de manière qu'une partie soit en contact électrique lorsque l'appareil est utilisé, caractérisé en ce qu'il comprend :
— au moins une poche sur l'extérieur du vêtement définie par le tissu du vêtement et un revêtement (8) en matériau fixé audit tissu sur sa périphérie, une électrode (10) étant disposée dans chaque poche en définissant un espace pour l'introduction d'une matière électrolytique ;
— la porosité du vêtement étant suffisante pour permettre à ladite matière fluide de pénétrer à travers de manière que la peau du patient soit mouillée par ladite matière fluide lorsque le vêtement est porté afin de compléter la connexion électrique entre l'électrode et la peau du patient.

2. Appareil sur vêtement suivant la revendication 1, caractérisé en ce que l'électrode a la configuration d'un disque.

3. Appareil sur vêtement suivant la revendication 2, caractérisé en ce que l'électrode est fixée le long de sa partie périphérique sur le vêtement.

4. Appareil sur vêtement suivant la revendication 2, caractérisé en ce que le conducteur de l'électricité adhère au vêtement.

5. Appareil sur vêtement suivant la revendication 4, caractérisé en ce que le conducteur de l'électricité est isolé.

6. Appareil sur vêtement suivant la revendication 5, caractérisé en ce que le conducteur a la forme d'un ruban souple.

7. Appareil sur vêtement suivant la revendication 6, caractérisé en ce que le conducteur adhère au vêtement par couture sur celui-ci.

8. Appareil sur vêtement suivant la revendication 7, caractérisé en ce que l'électrode est faite de la même matière que le conducteur.

9. Appareil sur vêtement suivant la revendication 8, caractérisé en ce que le conducteur établit un contact série avec une pluralité d'électrodes.

10. Appareil sur vêtement suivant la revendication 1, caractérisé en ce que le vêtement est fait d'une matière tissée.

11. Appareil suivant la revendication 1, caractérisé en ce que le vêtement est fait d'une matière non tissée.

12. Appareil selon la revendication 1, caractérisé en ce que la poche est formée par un morceau de matière (8) à l'épreuve du fluide conducteur et présentant un orifice (12) et recevant un chapeau de fermeture (13).

13. Appareil selon la revendication 1, caractérisé en ce que la poche est fermée et reçoit intérieurement une capsule (9) comprenant un fluide conducteur comprimé entre le vêtement (1) et l'électrode (10) ou entre l'électrode (10) et le revêtement (8).

14. Appareil selon la revendication 1, caractérisé en ce que la poche (6) avec orifice d'application (7) de la matière fluide est combinée avec un cylindre tubulaire (17).

15. Appareil selon la revendication 1, caractérisé en ce que le ou les conducteurs entre les électrodes sont construits à partir d'une fine bande de tissu souple conductrice électriquement qui est recouverte d'un tissu isolant réalisé dans le même matériau que le vêtement.

16. Appareil selon la revendication 1, caractérisé en ce que la poche (6) est réalisée en une matière conductrice.

17. Appareil selon la revendication 1, caractérisé en ce que l'électrode est en contact avec le revêtement (8).

18. Appareil selon la revendication 1, caractérisé en ce que l'électrode est en contact avec le vêtement.

19. Appareil selon la revendication 1, caractérisé en ce qu'un morceau de tissu (16) est placé entre l'électrode (10) et le vêtement (1), la poche étant formée entre l'électrode (10) et le tissu (16).

20. Appareil selon la revendication 1, caractérisé en ce que les conducteurs (2) sont disposés dans un tissu non conducteur fixé par couture à la surface extérieure du vêtement.

## Claims

1. Unit equipping an article of clothing in order to transmit or take electric impulses comprising :
— an article of clothing (1) made out of an electrically non conductive porous fabric and externally taking a plurality of electrcdes connected by means of conductors (2) fixed on the outside of the article of clothing so as to cover part of it ;
— one end of the conductors being in selective electric contact with an electric impulse transmitter or an electric sensor, the conductors being positioned with respect to the electrodes so that part is in electric contact when the unit is used, wherein it comprises :
— at least one pocket on the outside of the article of clothing defined by the fabric of the clothing and a coating (8) made of material fixed to the said fabric on its periphery, an electrode (10) being arranged in each pocked defining a space for the introduction of an electrolytic material ;
— the article of clothing being porous enough to enable the said fluid material to penetrate so that the skin of the patient is moistened by the said fluid when the article of clothing is worn in order to complete the electrical connection between the electrode and the skin of the patient.

2. Unit on an article of clothing according to claim 1, wherein the electrode is in the configuration of a disk.

3. Unit on an article of clothing according to claim 2, wherein the electrode is fixed along its peripheral part on the article of clothing.

4. Unit on an article of clothing according to claim 2, wherein the conductor of electricity adheres to the article of clothing.

5. Unit on an article of clothing according to claim 4, wherein the conductor of electricity is insulated.

6. Unit on an article of clothing according to claim 5, wherein the conductor is in the form of a flexible tape.

7. Unit on an article of clothing according to claim 6, wherein the conductor adheres to the article of clothing by sewing it on.

8. Unit on an article of clothing according to claim 7, wherein the electrode is made of the same material as the conductor.

9. Unit on an article of clothing according to claim 8, wherein the conductor makes series contact with a plurality of electrodes.

10. Unit on an article of clothing according to claim 1, wherein the article of clothing is made of a woven material.

11. Unit according to claim 1, wherein the article of clothing is made of a non-woven material.

12. Unit according to claim 1, wherein the pocket is formed by a piece of material (8) resistant to the conductor fluid and having an opening (12) and taking a closing cap (13).

13. Unit according to claim 1, wherein the pocket is closed and internally takes a cap (9) comprising a conductor fluid compressed between the article of clothing (1) and the electrode (10) or between the electrode (10) and the coating (8).

14. Unit according to claim 1, wherein the pocket (6) with opening (7) to introduce the fluid is combined with a tubular cylinder (17).

15. Unit according to claim 1, wherein the conductor(s) between the electrodes are made of a thin strip of flexible electrically conductive fabric which is covered with an insulating fabric made of the same material as the article of clothing.

16. Unit according to claim 1, wherein the pocket (6) is made of a conductive material.

17. Unit according to claim 1, wherein the electrode is in contact with the coating (8).

18. Unit according to claim 1, wherein the electrode is in contact with the article of clothing.

19. Unit according to claim 1, wherein a piece of fabric (16) is placed between the electrode (10) and the article of clothing (1), the pocket being formed between the electrode (10) and the fabric (16).

20. Unit according to claim 1, wherein the conductors (2) are arranged in a non conductive fabric fixed by sewing to the external surface of the article of clothing.


**Patentansprüche**

1. Gerät auf einem Kleidungsstück zur Abgabe oder Aufnahme von elektrischen Impulsen bestehend aus :

— einer Kleidungsstück (1) aus einem porigen, nicht elektrizitätsleitenden Stoff, und außen mit einer Mehrzahl Elektroden ausgerüstet, die durch im Innern der Kleidung befestigte Leiter (2) verbunden werden, so daß ein Teil des Kleidungsstücks dadurch bedeckt ist ;

— einem Leiter-Endstück in selektiv elektrischem Kontakt mit einem elektrischen Impulsgeber oder einem elektrischen Aufnehmer, wobei die Leiter mit Bezug auf die Elektroden so positioniert sind daß ein Teil bei Benutzung des Gerätes sich in elektrischem Kontakt befindet, dadurch gekennzeichnet daß, es besteht aus :

— mindestens einer Tasche auf der Kleidungsstücks-Außenseite wobei die Tasche besteht aus dem Kleidungsstoff und einem Überzug (8) aus einem Stoff der auf den Umfang des Bekleidungsstoffes befestigt wird, einer Elektrode (10) die jeweils in jeder Tasche angeordnet ist, wobei ein Raum zur Aufnahme eines elektrolytischen Stoffs vorgesehen wird ;

— die Porosität des Bekleidungsstückes is groß genug um den Zufluß der Flüssigkeit durch den Stoff zu gestatten, damit die Haut des Patienten durch die genannte Flüssigkeit beim Tragen der Kleidung angefeuchtet wird um die elektrische Verbindung zwischen der Elektrode und der Haut des Patienten zu vervollständigen.

2. Gerät auf einem Kleidungsstück nach Anspruch 1, dadurch gekennzeichnet daß die Elektrode die Form einer Scheibe hat.

3. Gerät auf einem Kleidungsstück nach Anspruch 2, dadurch gekennzeichnet daß die Elektrode auf dem Kleidungsstück längs des Umfangs befestigt ist.

4. Gerät auf einem Kleidungsstück nach Anspruch 2, dadurch gekennzeichnet daß der Elektrizitätsleiter auf dem Kleidungsstück haftet.

5. Gerät auf einem Kleidungsstück nach Anspruch 4, dadurch gekennzeichnet daß der Elektrizitätsleiter isoliert ist.

6. Gerät auf einem Kleidungsstück nach Anspruch 5, dadurch gekennzeichnet daß der Elektrizitätsleiter die Form eines flexiblen Bandes aufweist.

7. Gerät auf einem Kleidungsstück nach Anspruch 6, dadurch gekennzeichnet daß der Elektrizitätsleiter auf dem Kleidungsstück durch Nähen befestigt ist.

8. Gerät auf einem Kleidungsstück nach Anspruch 7, dadurch gekennzeichnet daß die Elektrode aus dem gleichen Stoff als der Leiter besteht.

9. Gerät auf einem Kleidungsstück nach Anspruch 8, dadurch gekennzeichnet daß der Leiter einen Serienkontakt mit mehreren Elektroden herstellt.

10. Gerät auf einem Kleidungsstück nach Anspruch 1, dadurch gekennzeichnet daß das Kleidungsstück aus einem gewebten Stoff besteht.

11. Gerät nach Anspruch 1, dadurch gekennzeichnet daß das Kleidungsstück aus einem nichtgewebten Stoff besteht.

12. Gerät nach Anspruch 1, dadurch gekennzeichnet daß die Tasche aus einem Stück Stoff (8) besteht der gegenüber der leitenden Flüssigkeit widerstandsfähig ist und mit einer Öffnung versehen ist (12) auf welcher eine Abschlußkappe (13) vorgesehen wird.

13. Gerät nach Anspruch 1, dadurch gekennzeichnet daß die Tasche geschlossen ist, und enthält eine innere Kapsel (9) mit leitender Flüssigkeit, die zwischen dem Kleidungsstück (1) und der Elektrode (10) bzw. zwischen der Elektrode (10) und dem Überzug (8) gepresst wird.

14. Gerät nach Anspruch 1, dadurch gekennzeichnet daß die Tasche (6) mit Mündung (7) zum Einfüllen der Flüssigkeit mit einem rohrförmigen Zylinder (17) verbunden ist.

15. Gerät nach Anspruch 1, dadurch gekennzeichnet daß der bzw. die Leiter zwischen den Elektroden aus einem elektrisch leitenden dünnen flexiblen Stoffstreifen mit Isolierstoffüberzug hergestellt werden, der aus dem gleichen Stoff als das Kleidungsstück gefertigt ist.

16. Gerät nach Anspruch 1, dadurch gekennzeichnet daß die Tasche (6) aus einem leitenden Stoff hergestellt wird.

17. Gerät nach Anspruch 1, dadurch gekennzeichnet daß die Elektrode mit dem Überzug (8) in Kontakt ist.

18. Gerät nach Anspruch 1, dadurch gekennzeichnet daß die Elektrode mit dem Kleidungsstück in Kontakt ist.

19. Gerät nach Anspruch 1, dadurch gekennzeichnet daß ein Stück Stoff (16) zwischen der Elektrode (10) und dem Kleidungsstück (1) eingesetzt ist, wobei die Tasche zwischen der Elektrode (10) und dem Stoff (16) geformt wird.

20. Gerät nach Anspruch 1, dadurch gekennzeichnet daß die Leiter (2) in einem nichtleitenden Stoff angeordnet sind, der durch Nähen auf die Außenoberfläche des Kleidungsstücks befestigt wird.

FIG.1

FIG.2

FIG.3

FIG.4

FIG.5

FIG.6

FIG.7

FIG.8

FIG.9

FIG.10